(19) Europäisches Patentamt / European Patent Office / Office européen des brevets

(11) **EP 1 905 453 B1**

(12) **EUROPEAN PATENT SPECIFICATION**

(45) Date of publication and mention
of the grant of the patent:
**31.10.2012 Bulletin 2012/44**

(51) Int Cl.:
*A61K 9/00* *(2006.01)*  *A61K 47/04* *(2006.01)*
*A61K 47/10* *(2006.01)*  *A61K 47/26* *(2006.01)*
*A61K 47/34* *(2006.01)*  *A61K 47/18* *(2006.01)*
*A61K 47/22* *(2006.01)*

(21) Application number: **06781064.8**

(22) Date of filing: **13.07.2006**

(86) International application number:
**PCT/JP2006/313944**

(87) International publication number:
**WO 2007/007832 (18.01.2007 Gazette 2007/03)**

(54) **PRESERVATIVE COMPOSITION FOR OPHTHALMIC USE**

KONSERVIERUNGSMITTEL ZUR OPHTHALMISCHEN ANWENDUNG

COMPOSITION CONSERVATRICE À USAGE OPHTALMIQUE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **13.07.2005 JP 2005204472**

(43) Date of publication of application:
**02.04.2008 Bulletin 2008/14**

(73) Proprietor: **SANTEN PHARMACEUTICAL CO.,
LTD.
Higashiyodogawa-ku,
Osaka-shi,
Osaka 533-8651 (JP)**

(72) Inventors:
• **KIMURA, Akio**
  **Osaka-shi, Osaka 533-8651 (JP)**
• **TAKADA, Koichi**
  **Osaka-shi, Osaka 533-8651 (JP)**
• **OKEMOTO, Mikiko**
  **Osaka-shi, Osaka 533-8651 (JP)**

(74) Representative: **Müller-Boré & Partner
Patentanwälte
Grafinger Straße 2
81671 München (DE)**

(56) References cited:
**EP-A2- 0 196 075       WO-A1-94/13332
WO-A1-99/43290        US-A- 5 648 074
US-A1- 2004 175 435**

**Description**

The present invention relates to a preservative composition for ophthalmic use comprising a chlorite and a stabilizer for the chlorite, and a liquid preparation for ophthalmic use comprising the composition.

Eye drops are directly administered to a sensitive organ of the human body called eyes, and further, contact lenses are also used in a situation in which they are directly contacted with the eyes. Therefore, attention should be paid to the components to be formulated in eye drops or saline solutions for contact lenses from the viewpoint of safety. In particular, irritation to the eyes, side effects and the like should be taken into consideration.

As a preservative component of eye drops, for example, benzalkonium chloride, benzethonium chloride, sorbic acid and the like, and as a preservative component of saline solutions for contact lenses, for example, polyhexamethylene biguanide (PHMB), Polyquad, hydrogen peroxide, Purite (stabilized chlorine dioxide) and the like have been put to practical use.

Benzalkonium chloride or benzethonium chloride has an excellent preservative effect, however, when its concentration is increased, a corneal disorder may be sometimes caused. Therefore, its concentration when used is restricted to a certain degree. Further, these preservatives may sometimes cause alteration of the effects due to chemical reaction with an acidic additive, and also have a property to be easily adsorbed on eye drop containers or filtration filters. Sorbic acid is less likely to be adsorbed on eye drop containers, however, its preservative effect is not sufficient, and further, its stability is decreased depending on the pH, therefore, the formulation thereof in eye drops is restricted to a certain degree. On the other hand, peroxide preservatives such as hydrogen peroxide exhibit excellent disinfecting and washing effects when they are formulated in saline solutions for contact lenses, however, they are required to be neutralized because of their irritativeness.

A chlorite is a compound having $ClO_2^-$ ion, and particularly sodium chlorite is used as a disinfectant for tap water or the like. However, it is known that when a chlorite is decomposed, chlorine dioxide having a strong oxidizing action is generated to cause irritation to the eyes, skin, respiratory tract, etc. On the other hand, JP-A-3-164402 discloses an invention relating to a method for producing chlorine dioxide and a disinfectant composition, in which chlorine dioxide is generated from a chlorine dioxide precursor such as a chlorite using a transition metal and thereby contact lenses and the like are disinfected and washed taking advantage of the strong disinfecting action of chlorine dioxide.

US-patent 5,648,074 describes compositions and methods for disinfecting contact lenses and reducing proteinaceous deposit formation. US 2004/075435 A1 describes preserved cyclodextrin-containing compositions. EP 0 196 075 A2 describes a cleaning system for contact lenses and a process for cleaning the same.

Chlorine dioxide is a strong oxidizing agent like hydrogen peroxide and is known to have high irritativeness to the eyes, skin, etc. Therefore, when a chlorite is used as a preservative component for ophthalmic use, it is an important subject to prevent the generation of chlorine dioxide thereby to increase safety.

The present inventors studied stabilizers for preventing the generation of chlorine dioxide in various manners, and as a result, they found that a liquid preparation for ophthalmic use containing a preservative composition for ophthalmic use comprising a chlorite as a preservative component, and at least one stabilizer selected from creatinine, geraniol, oxyquinoline sulfate and polysorbate 80 can significantly prevent the generation of chlorine dioxide, and is therefore excellent in safety and exhibits a sustained preservative

effect for a prolonged period of time.

That is, the present invention is directed to:

(1) a preservative composition for ophthalmic use comprising a chlorite and at least one stabilizer selected from the following 1) to 4):

1) creatinine;
2) geraniol;
3) oxyquinoline sulfate; and
4) polysorbate 80;

(2) the preservative composition for ophthalmic use acording to the above (1) further comprising tocopherol acetate, wherein the stabilizer is polysorbate 80;

(3) the preservative composition for ophthalmic use according to the above (1) or (2), wherein the chlorite is sodium chlorite;

(4) a liquid preparation for ophthalmic use comprising the preservative composition for ophthalmic use according to any one of the above (1) to (3);

(5) the liquid preparation for ophthalmic use according to the above (4), wherein the preparation comprises 0.00001 to 1% (w/v) of a chlorite and at least one stabilizer selected from the following 1) to 4):

1) 0.0001 to 5% (w/v) of creatinine;
2) 0.00001 to 0.05% (w/v) of geraniol;
3) 0.00001 to 1% (w/v) of oxyquinoline sulfate; and
4) 0.0001 to 10% (w/v) of polysorbate 80; and

(6) the liquid preparation for ophthalmic use according to the above (4), wherein the preparation comprises 0.00001 to 1% (w/v) of a chlorite and a stabilizer which is a mixture of 0.0001 to 1% (w/v) of tocopherol acetate and 0.0001 to 10% (w/v) of polysorbate 80.

In the present invention, the chlorite as a preservative component is not particularly limited as long as it is a salt of chlorous acid, and examples thereof include alkali metal salts of chlorous acid such as sodium chlorite and potassium chlorite, alkaline earth metal salts of chlorous acid such as calcium chlorite, magnesium chlorite and barium chlorite, copper chlorite, lead chlorite, ammonium chlorite and the like, and more preferred chlorite is sodium chlorite.

The concentration of the chlorite in the liquid preparation for ophthalmic use is preferably in the range of from 0.00001 to 1 % (w/v), more preferably from 0.0001% to 0.1 % (w/v).

In the present invention, examples of the stabilizer capable of stabilizing the chlorite as a preservative component thereby to prevent the generation of chlorine dioxide include the following 5 substances.

1) creatinine
2) geraniol
3) tocopherol acetate
4) oxyquinoline sulfate
5) polysorbate 80

The concentration of the stabilizer in the liquid preparation for ophthalmic use is preferably in the range of from 0.0001 to 5% (w/v) if it is creatinine, from 0.00001 to 0.05% (w/v) if it is geraniol, from 0.0001 to 1% (w/v) if it is tocopherol acetate ($\alpha$, $\beta$, $\gamma$, $\delta$), from 0.00001 to 1% (w/v) if it is oxyquinoline sulfate, from 0.0001 to 10% (w/v) if it is polysorbate 80.

The stabilisers creatinine, geraniol, oxyquinoline sulfate and polysorbate 80 may be used alone or in combination thereof, and tocopherol acetate is used in combination with polysorbate 80.

The liquid preparation for ophthalmic use of the present invention is preferably used as, for example, an eye drop or a saline solution for contact lenses, and can be prepared by widely used methods.

Further, in the liquid preparation for ophthalmic use of the present invention, a drug, a tonicity agent, a buffer, a pH adjusting agent, a viscosity incresing agent or the like can be appropriately formulated as needed.

The drug to be formulated in the liquid preparation for ophthalmic use of the present invention is not particularly limited, and examples thereof include antiglaucoma agents (such as timolol, prostaglandin derivatives and carbonate dehydratase inhibitors), a variety of vitamins (such as vitamin B2, vitamin B6, vitamin B12, vitamin E and panthenol), decongestants (such as tetrahydrozoline hydrochloride and naphazoline hydrochloride), antiinflammatory drugs (such as diclofenac, indometacin, fluorometholone, pranoprofen, glycyrrhizinate dipotassium and $\epsilon$ -aminocaproic acid), anti-histamines (such as chlorpheniramine maleate and diphenhydramine hydrochloride), antiallergic drugs (such as sodium cromoglicate), antimicrobial drugs (such as quinolone antimicrobial agents, cephalosporins, sulfacetamide sodium and sulfamethoxazole), amino acids (such as potassium L-aspartate, aminoethylsulfonic acid and sodium chondroitin sulfate), diagnostic reagents (such as fluorescein sodium), sodium hyaluronate, neostigmine methylsulfate and the like.

Examples of the tonicity agent include glycerin, propylene glycol, polyethylene glycol, sodium chloride, potassium chloride, calcium chloride, magnesium chloride and the like.

Examples of the buffer include phosphates such as sodium phosphate, sodium dihydrogen phosphate, disodium hydrogen phosphate, potassium phosphate, potassium dihydrogen phosphate and dipotassium hydrogen phosphate; borates such as sodium borate and potassium borate; citrates such as sodium citrate and disodium citrate; acetates such as sodium acetate and potassium acetate; carbonates such as sodium carbonate, sodium hydrogen carbonate; trometamol and epsilon-aminocaproic acid, and the like.

Examples of the pH adjusting agent include hydrochloric acid, citric acid, phosphoric acid, acetic acid, sodium hydroxide, potassium hydroxide, sodium carbonate, sodium hydrogen carbonate and the like.

Examples of the viscosity incresing agent include hydroxypropylmethyl cellulose, hydroxypropyl cellulose, polyvinyl alcohol, carboxyvinyl polymer, polyvinylpyrrolidone and the like.

The pH of the liquid preparation for ophthalmic use of the present invention is preferably in the range of from 3 to 9, particularly from 5 to 8.

The liquid preparation for ophthalmic use containing the preservative composition for ophthalmic use comprising a chlorite and at least one stabilizer selected from creatinine, geraniol, oxyquinoline sulfate and polysorbate 80 can prevent the generation of chlorine dioxide, and is therefore excellent in safety and exhibits a sustained preservative

effect for a prolonged period of time.

1. Test for prevention of generation of chlorine dioxide

(1) Sample preparation

Test solution 1

7 mg of a chlorite, 500 mg of creatinine and 20 mg of sodium hydrogen phosphate were dissolved in about 80 mL of purified water, and the pH of the solution was adjusted to 7.0 with dilute hydrochloric acid or sodium hydroxide. Then, the total volume was made up to 100 mL with purified water, whereby Test solution 1 was obtained.

Test solutions 2 to 6

Test solutions 2 to 6, of which Test solutions 3 and 4 were Reference test solutions, were obtained by carrying out the same procedure as that of Test solution 1 except that 5 mg of geraniol, 200 mg of glucose, 2 g of mannitol, 100 mg of polysorbate 80 and 10 mg of oxyquinoline sulfate were used, respectively, in place of 500 mg of creatinine in Test solution 1.

Test solution 7

140 mg of d-$\alpha$-tocopherol acetate and 100 mg of polysorbate 80 were mixed, and 7 mg of a chlorite, 20 mg of sodium hydrogen phosphate and about 80 mL of purified water were added thereto and dissolved. Then, the pH of the solution was adjusted to 7.0 with dilute hydrochloric acid or sodium hydroxide, and the total volume was made up to 100 mL with purified water, whereby Test solution 7 was obtained.

Comparative test solution 1

7 mg of a chlorite and 20 mg of sodium hydrogen phosphate were dissolved in about 80 mL of purified water, and the pH of the solution was adjusted to 7.0 with dilute hydrochloric acid or sodium hydroxide. Then, the total volume was made up to 100 mL with purified water, whereby Comparative test solution 1 was obtained.

(2) Test method and results

1) Determination of chlorine dioxide by DPD method (for 14 days)

By using Test solutions 1 to 5 and Comparative test solution 1, the concentration (ppm) of generated chlorine dioxide was determined by the DPD method described in the Standard Methods for Examination of water. These test results are shown in Table 1.

[Table 1]

| | Stabilizer | Concentration of chlorine dioxide (ppm) | |
| --- | --- | --- | --- |
| | | After 7 days | After 14 days |
| Test solution 1 | Creatinine | N.D. *1 | N.D. |
| Test solution 2 | Geraniol | N.D. | N.D. |
| Test solution 3 | Glucose | N.D. | N.D. |
| Test solution 4 | Mannitol | N.D. | N.D. |
| Test solution 5 | Polysorbate 80 | N.D. | N.D. |
| Comparative test solution 1 | | 0.087 | 0.15 |
| Note) *1: N.D. indicates that the value is 0.02 ppm or less (measuring range of DPD method: 0.02 to 5 ppm). | | | |

2) Confirmation of existence of chlorine dioxide with detector tube (for 14 days)

Determination by the DPD method could not be carried out for Test solutions 6 and 7 because they were colored, therefore, the existence of chlorine dioxide was confirmed using a simple test method with a detector tube. To be more specific, chlorine dioxide in Test solutions 6 and 7 and Comparative test solution 1 was vaporized under reduced pressure, and the existence of chlorine dioxide was confirmed with a detector tube. These test results are shown in Table 2.

[Table 2]

| | Stabilizer | Concentration of chlorine dioxide after 14 days (ppm) |
|---|---|---|
| Test solution 6 | Oxyquinoline sulfate | N.D.[*2] |
| Test solution 7 | D-$\alpha$-tocopherol acetate and Polysorbate 80 | N.D. |
| Comparative test solution 1 | | 0.2 |
| | | |
| Note) *2: N. D. indicates that the existence of chlorine dioxide was not observed (measuring range of detector tube: 0.05 to 0.6 ppm). | | |

(3) Discussion

As apparent from Table 1 and Table 2, the liquid preparations for ophthalmic use containing the preservative composition for ophthalmic use comprising a chlorite and each stabilizer (creatinine, geraniol, glucose, mannitol, polysorbate 80, oxyquinoline sulfate and d-$\alpha$-tocopherol acetate) can significantly prevent the generation of chlorine dioxide and are therefore excellent in safety and cause less irritation to the eyes.

2. Test for preservative effectiveness

(1) Test for preservative effectiveness using P. aeruginosa and C. albicans

1) Sample

The above-mentioned Test solutions 1 to 7 were used.

2) Test method and results

A test for preservative effectiveness was carried out according to Preservatives-Effectiveness Tests described in the Japanese Pharmacopoeia Fourteenth Edition. As test microorganisms, Pseudomonas aeruginosa (P. aeruginosa) and Candida albicans (C. albicans) were used, and the viable cell numbers after 7 days and 14 days were measured. The survival rate (%) of the microorganisms was calculated according to the following calculation equation.

```
Survival rate (%) = [(Viable cell number when sampling) /

(Initial cell number)] x 100
```

These test results are shown in Table 3.

[Table 3]

| | Stabilizer | Test microorganism | Survival rate (%) | |
|---|---|---|---|---|
| | | | After 7 days | After 14 days |
| Test solution 1 | Creatinine | P. aeruginosa | N.D. [*3] | N.D. |
| | | C. albicans | 20.7 | 10.0 |

(continued)

| | | Stabilizer | Test microorganism | Survival rate (%) | |
|---|---|---|---|---|---|
| | | | | After 7 days | After 14 days |
| Test solution 2 | | Geraniol | P. aeruginosa | N.D. | N.D. |
| | | | C. albicans | 3.3 | 0.5 |
| Test solution 3 | | Glucose | P. aeruginosa | N.D. | N.D. |
| | | | C. albicans | N.D. | N.D. |
| Test solution 4 | | Mannitol | P. aeruginosa | N.D. | N.D. |
| | | | C. albicans | 65.3 | 54.0 |
| Test solution 5 | | Polysorbate 80 | P. aeruginosa | N.D. | N.D. |
| | | | C. albicans | 66.7 | 66.7 |
| Test solution 6 | | Oxyquinoline sulfate | P. aeruginosa | N.D. | N.D. |
| | | | C. albicans | 5.1 | 0.1 |
| Test solution 7 | D-$\alpha$-tocopherol acetate and Polysorbate 80 | | P. aeruginosa | N.D. | N.D. |
| | | | C. albicans | 58.7 | 17.3 |
| Note) *3: N.D. indicates that microorganisms were not detected. | | | | | |

(2) Test for preservative effectiveness using A. niger

1) Sample preparation

Test solution 8

20 mg of a chlorite, 5 mg of geraniol and 200 mg of sodium hydrogen phosphate were dissolved in purified water, and the pH of the solution was adjusted to 6.5 with dilute hydrochloric acid or sodium hydroxide. Then, the total volume was made up to 100 mL with purified water, whereby Test solution 8 was obtained.

Test solution 9 and Reference test solution 10

Test solution 9 and Reference test solution 10 were obtained by carrying out the same procedure as that of Test solution 8 except that 50 mg of creatinine and 2 g of mannitol were used, respectively, in place of 5 mg of geraniol in Test solution 8.

Test solutions 11 and 12

Test solutions 11 and 12 were obtained by carrying out the same procedure as that of Test solution 8 except that the amount of a chlorite was changed from 20 mg in Test solution 8 to 50 mg and 7 mg, respectively.

2) Test method and results

By using Test solutions 8 to 12, a test for preservative effectiveness was carried out. The test for preservative effectiveness was carried out according to Preservatives-Effectiveness Tests described in the Japanese Pharmacopoeia Fourteenth Edition. As a test microorganism, Aspergillus niger (A. niger) was used, and the viable cell numbers after 7 days, 14 days and 28 days were measured. The acceptance criteria of the Japanese Pharmacopoeia (JP) and the US Pharmacopoeia (USP) for Category IA into which eye drops are classified are shown below.
Acceptance criteria of JP (fungi): The viable cell numbers after 14 days and 28 days are the same as or lower than the inoculated cell number.
Acceptance criteria of USP (fungi): The viable cell numbers after 7 days, 14 days and 28 days do not increase from the inoculated cell number.
These test results are shown in Table 4.

[Table 4]

|  | Stabilizer | Chlorite (mg) | Test microorganism | Acceptance result (JP) | Acceptance result (USP) |
|---|---|---|---|---|---|
| Test solution 8 | Geraniol | 20 | A. niger | Accepted | Accepted |
| Test solution 9 | Creatinine | 20 | A. niger | Accepted | Accepted |
| Test solution 10 | Mannitol | 20 | A. niger | Accepted | Accepted |
| Test solution 11 | Geraniol | 50 | A. niger | Accepted | Accepted |
| Test solution 12 | Geraniol | 7 | A. niger | Accepted | Accepted |

(3) Test for preservative effectiveness using 5 species of microorganisms

1) Sample preparation

Test solution 13

7 mg of a chlorite, 5 mg of geraniol, 400 mg of potassium chloride, 100 mg of sodium chloride and 1 g of boric acid were dissolved in purified water, and the pH of the solution was adjusted to 7.5 with dilute hydrochloric acid or sodium hydroxide. Then, the total volume was made up to 100 mL with purified water, whereby Test solution 13 was obtained.

Reference test solution 14

Reference test solution 14 was obtained by carrying out the same procedure as that of Test solution 13 except that 2 g of mannitol was used in place of 5 mg of geraniol in Test solution 13. Comparative test solution 2

400 mg of potassium chloride, 100 mg of sodium chloride and 1 g of boric acid were dissolved in purified water, and the pH of the solution was adjusted to 7.5 with dilute hydrochloric acid or sodium hydroxide. Then, the total volume was made up to 100 mL with purified water, whereby Comparative test solution 2 was obtained.

2) Test method and results

By using Test solution 13, Reference test solution 14 and Comparative test solution 2, a test for preservative effectiveness was carried out. The test for preservative effectiveness was carried out according to Preservatives-Effectiveness Tests described in the Japanese Pharmacopoeia Fourteenth Edition. As test microorganisms, Escherichia coli (E. coli), Pseudomonas aeruginosa (P. aeruginosa), Staphylococcus aureus (S. aureus), Candida albicans (C. albicans) and Aspergillus niger (A. niger) were used, and the viable cell numbers after 14 days and 28 days were measured. The survival rate (%) of the microorganisms was calculated according to the following calculation equation.

```
Survival rate (%) = [(Viable cell number when sampling) /

(Initial cell number)] x 100
```

These test results are shown in Table 5.

[Table 5]

| | Stabilizer | Test microorganism | Survival rate (%) | |
|---|---|---|---|---|
| | | | After 14 days | After 28 days |
| Test solution 13 | Geraniol | E. coli | 0.0 | 0.0 |
| | | P. aeruginosa | 0.0 | 0.0 |
| | | S. aureus | 0.0 | 0.0 |
| | | C. albicans | 0.9 | 0.0 |
| | | A. niger | 0.6 | 0.5 |
| Test solution 14 - | Mannitol | E. coli | 0.0 | 0.0 |
| | | P. aeruginosa | 0.0 | 0.0 |
| | | S. aureus | 0.0 | 0.0 |
| | | C. albicans | 0.0 | 0.0 |
| | | A. niger | 0.1 | 0.1 |
| Comparative test solution 2 | | E. coli | 123.1 | 92.3 |
| | | P. aeruginosa | 0.4 | 0.1 |
| | | S. aureus | 0.8 | 0.0 |
| | | C. albicans | 47.4 | 0.0 |
| | | A. niger | 0.7 | 2.6 |

(4) Discussion

As apparent from Tables 3 to 5, the liquid preparations for ophthalmic use containing the preservative composition for ophthalmic use comprising a chlorite and each of the above-mentioned stabilizers exhibit an excellent preservative effect on various microorganisms such as Pseudomonas aeruginosa (gram-negative bacterium), Caidida (fungus) and Aspergillus niger (fungus).

3. Preparation examples

Hereinafter, typical preparation examples will be shown. Formulation example 1 (pH 7)
In 100 ml

| | |
|---|---|
| Sodium hyaluronate | 100 mg |
| Sodium chlorite | 7 mg |
| Creatinine | 50 mg |
| Sodium chloride | 850 mg |
| Sodium dihydrogen phosphate | 200 mg |
| Sodium hydroxide | q.s. |
| Dilute hydrochloric acid | q.s. |
| Sterile purified water | q.s. |

Formulation example 2 (pH 7)

In 100 ml

| | |
|---|---|
| Sodium hyaluronate | 100 mg |
| Sodium chlorite | 7 mg |
| Geraniol | 5 mg |
| Sodium chloride | 900 mg |

(continued)

| Sodium dihydrogen phosphate | 200 mg |
| Sodium hydroxide | q.s. |
| Dilute hydrochloric acid | q.s. |
| Sterile purified water | q.s. |

Formulation example 3 (pH 7) as a reference

In 100 ml

| Sodium hyaluronate | 100 mg |
| Sodium chlorite | 7 mg |
| Mannitol | 2 g |
| Sodium dihydrogen phosphate | 200 mg |
| Sodium hydroxide | q.s. |
| Dilute hydrochloric acid | q.s. |
| Sterile purified water | q.s. |

Formulation example 4 (pH 7)

In 100 ml

| Potassium chloride | 100 mg |
| Sodium chloride | 400 mg |
| Sodium chlorite | 7 mg |
| Geraniol | 5 mg |
| Boric acid | 1 g |
| Sodium hydroxide | q.s. |
| Dilute hydrochloric acid | q.s. |
| Sterile purified water | q.s. |

**Claims**

1. A preservative composition for ophthalmic use comprising a chlorite and at least one stabilizer selected from the following 1) to 4):

   1) creatinine;
   2) geraniol;
   3) oxyquinoline sulfate; and
   4) polysorbate 80.

2. The preservative composition for ophthalmic use acording to claim 1 further comprising tocopherol acetate, wherein the stabilizer is polysorbate 80.

3. The preservative composition for ophthalmic use according to claim 1 or 2, wherein the chlorite is sodium chlorite.

4. A liquid preparation for ophthalmic use comprising the preservative composition for ophthalmic use according to any one of claims 1 to 3.

5. The liquid preparation for ophthalmic use according to claim 4, wherein the preparation comprises 0.00001 to 1 % (w/v) of a chlorite and at least one stabilizer selected from the following 1) to 4):

   1) 0.0001 to 5% (w/v) of creatinine;

2) 0.00001 to 0.05% (w/v) of geraniol;
3) 0.00001 to 1% (w/v) of oxyquinoline sulfate; and
4) 0.0001 to 10% (w/v) of polysorbate 80.

6. The liquid preparation for ophthalmic use according to claim 4, wherein the preparation comprises 0.00001 to 1% (w/v) of a chlorite and a stabilizer which is a mixture of 0.0001 to 1% (w/v) of tocopherol acetate and 0.0001 to 10% (w/v) of polysorbate 80.

**Patentansprüche**

1. Konservierungszusammensetzung zur ophthalmischen Anwendung, umfassend ein Chlorit und mindestens einen Stabilisator, ausgewählt aus den folgenden 1) bis 4):

   1) Kreatinin;
   2) Geraniol;
   3) Hydroxychinolinsulfat; und
   4) Polysorbat 80.

2. Konservierungszusammensetzung zur ophthalmischen Anwendung nach Anspruch 1, weiter umfassend Tocopherolacetat, wobei der Stabilisator Polysorbat 80 ist.

3. Konservierungszusammensetzung zur ophthalmischen Anwendung nach Anspruch 1 oder 2, wobei das Chlorit Natriumchlorit ist.

4. Flüssige Zubereitung zur ophthalmischen Anwendung, umfassend die Konservierungszusammensetzung zur ophthalmischen Anwendung nach einem der Ansprüche 1 bis 3.

5. Flüssige Zubereitung zur ophthalmischen Anwendung nach Anspruch 4, wobei die Zubereitung 0,00001 bis 1 % (w/v) eines Chlorits und mindestens einen Stabilisator, ausgewählt aus den folgenden 1) bis 4), umfasst:

   1) 0,0001 bis 5% (w/v) Kreatinin;
   2) 0,00001 bis 0.05% (w/v) Geraniol;
   3) 0,00001 bis 1 % (w/v) Hydroxychinolinsulfat; und
   4) 0,0001 bis 10% (w/v) Polysorbat 80.

6. Flüssige Zubereitung zur ophthalmischen Anwendung nach Anspruch 4, wobei die Zubereitung 0,00001 bis 1% (w/v) eines Chlorits und einen Stabilisator, der ein Gemisch aus 0,0001 bis 1% (w/v) Tocopherolacetat und 0,0001 bis 10% (w/v) Polysorbat 80 ist, umfasst.

**Revendications**

1. Composition de conservation pour usage ophtalmique, comprenant un chlorite et au moins un stabilisant choisi parmi les suivants 1) à 4) :

   1) créatinine ;
   2) géraniol ;
   3) sulfate d'oxyquinoléine, et
   4) polysorbate 80.

2. Composition de conservation pour usage ophtalmique selon la revendication 1, comprenant en outre l'acétate de tocophérol, où le stabilisant est le polysorbate 80.

3. Composition de conservation pour usage ophtalmique selon la revendication 1 ou 2, où le chlorite est le chlorite de sodium.

4. Préparation liquide pour usage ophtalmique comprenant la composition de conservation pour usage ophtalmique selon l'une quelconque des revendications 1 à 3.

5. Préparation liquide pour usage ophtalmique selon la revendication 4, où la préparation comprend 0,00001 à 1% (p/v) d'un chlorite et au moins un stabilisant choisi parmi les suivants 1) à 4) :

    1) 0,0001 à 5% (p/v) de créatinine ;
    2) 0,00001 à 0,05% (p/v) de géraniol ;
    3) 0,00001 à 1% (p/v) de sulfate d'oxyquinoléine, et
    4) 0,0001 à 10% (p/v) de polysorbate 80.

6. Préparation liquide pour usage ophtalmique selon la revendication 4, où la préparation comprend 0,00001 à 1% (p/v) d'un chlorite et un stabilisant qui est un mélange de 0,0001 à 1% (p/v) d'acétate de tocophérol et 0,0001 à 10% (p/v) de polysorbate 80.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3164402 A
- US 5648074 A
- US 2004075435 A1
- EP 0196075 A2